# FASCICULE DE BREVET EUROPEEN

(11) **EP 3 294 421 B1**
(45) Date de publication et mention de la délivrance du brevet: **11.08.2021**
(21) Numéro de dépôt: 16731210.7
(22) Date de dépôt: 13.05.2016
(51) Int. Cl.: A61Q 19/00, A61Q 19/08, A61H 33/06, A61H 33/12, A45D 44/00, B05B 7/14, B05B 17/04

(54) **METHODE DE TRAITEMENT COSMETIQUE DE LA PEAU**
VERFAHREN ZUR KOSMETISCHEN BEHANDLUNG DER HAUT
METHOD FOR COSMETIC TREATMENT OF THE SKIN

(30) Priorité: 13.05.2015 FR 1554338
(43) Date de publication de la demande: 21.03.2018
(73) Titulaire: Cha, Ling, 75008 Paris (FR)
(72) Inventeur: BOILLOT, Laurent, 92200 Neuilly Sur Seine (FR)
(74) Mandataire: Plasseraud IP
(86) Numéro de dépôt international: PCT/FR2016/051136
(87) Numéro de publication internationale: WO 2016/181086

(56) Documents cités:
- EP-A1- 2 377 421
- US-A1- 2006 213 005
- US-A1- 2011 166 089

## Description

La présente invention a pour objet une méthode de traitement cosmétique de la peau comprenant une pulvérisation d'au moins une molécule choisie parmi une molécule non volatile et une molécule instable à la température.

Les méthodes de traitement cosmétique de la peau, en particulier du visage, mettent classiquement en œuvre des formulations comprenant diverses molécules cosmétiquement actives destinées à être appliquées sur la peau. Les molécules actives pénètrent alors dans les couches supérieures de l'épiderme pour produire leur action cosmétique. Toutefois, la pénétration des molécules actives est souvent limitée par la structure intrinsèque de la peau constituée d'une succession de couches plus ou moins perméables.

Par ailleurs, l'application répétée de formulations cosmétiques comprenant des pigments ou des charges sur la peau, telles que des compositions de maquillage, peut obstruer les pores de la peau, freinant encore la pénétration des molécules actives.

Différents dispositifs pour le nettoyage de la peau ont été proposés pour désobstruer les pores, par exemple au moyen de brosses montées sur des têtes rotatives. De tels dispositifs peuvent toutefois s'avérer irritants pour la peau. Des méthodes plus douces à base de vapeur d'eau ont été récemment développées et rencontrent un important succès auprès des consommatrices. Ces méthodes reposent sur un effet « sauna » exercé par la vapeur d'eau sur la peau, générant une dilatation des pores et une sudation du fait du chauffage, permettant l'élimination des poussières, et des résidus de corps gras ou de particules accumulés dans les pores de la peau.

Les dispositifs de saunas faciaux disponibles sur le marché mettent en œuvre un courant de vapeur chaude destiné à être dirigé vers la peau de l'utilisatrice, notamment vers son visage, pour en permettre le nettoyage.

La demande FR-A-2 462 158 de la société Matsushita Electric Works décrit par exemple un dispositif de soin de la peau capable de chauffer la peau, mais également de la refroidir et de la laver, par le biais d'un moyen de pulvérisation d'eau pour générer un courant d'air humidifié, et d'un moyen pour chauffer au moins une partie dudit courant d'air humidifié.

L'utilisation de ce type de dispositif ne permet toutefois pas la diffusion de molécules actives à la surface de la peau.

Le brevet EP-B-2194950 de Danakim Investment Limited décrit quant à lui une méthode de traitement de la peau mettant en œuvre un premier cycle de chauffage et de vaporisation sur la peau d'une première composition traitante comprenant un agent de traitement, et un second cycle consécutif de chauffage et de vaporisation d'une seconde composition astreignante. Toutefois, les molécules non volatiles ne peuvent pas être véhiculées par la vapeur produite par ce type de dispositif. En outre, la température nécessaire à la formation d'un courant de vapeur d'eau à 100°C rend difficile voire impossible le transport de molécules actives dont la structure ne serait pas parfaitement stable à la chaleur.

D'autres documents représentant l'état de la technique sont US2011/166089 A1, US 2006/213005 A1, EP2377421 A1.

Il existe donc un besoin pour parvenir à véhiculer, au niveau de la peau, tout type de molécules actives, incluant les molécules non volatiles ou instables à la chaleur.

L'invention vise à répondre à ce besoin.

Ainsi, la présente invention a pour objet, selon un premier aspect, une méthode de traitement cosmétique de la peau comprenant une pulvérisation sur la peau d'au moins une molécule choisie parmi une molécule non volatile et une molécule instable à la température au moyen d'un dispositif de pulvérisation comprenant :
i. un système de vaporisation adapté pour vaporiser une composition comprenant de l'eau, et
ii. un système de nébulisation adapté pour nébuliser une composition comprenant au moins une molécule choisie parmi une molécule non volatile et une molécule instable à la température,
la méthode de traitement cosmétique prévoyant de vaporiser la composition comprenant de l'eau par le système de vaporisation et de nébuliser la composition comprenant au moins une molécule choisie parmi une molécule non volatile et une molécule instable à la température par le système de nébulisation.

Ainsi l'invention permet de bénéficier d'une ouverture et d'une désobstruction des pores obtenues par un courant de vapeur chaude résultant d'un effet sauna de la vaporisation, sans dégrader les molécules actives, incluant les molécules non volatiles ou instables à la chaleur, afin de favoriser leur action cosmétique.

Il est, par ailleurs, possible d'associer des moyens de séchage de la peau au dispositif de pulvérisation de molécules non volatiles et/ou instables à la température. Ces moyens peuvent être mis en œuvre postérieurement à une étape de pulvérisation de molécules non volatiles et/ou instables à la température.

D'autres objets et avantages de l'invention apparaîtront à la lecture de la description qui suit de modes de réalisation particuliers de l'invention donnés à titre d'exemple non limitatif, la description étant faite en référence aux dessins annexés dans lesquels :

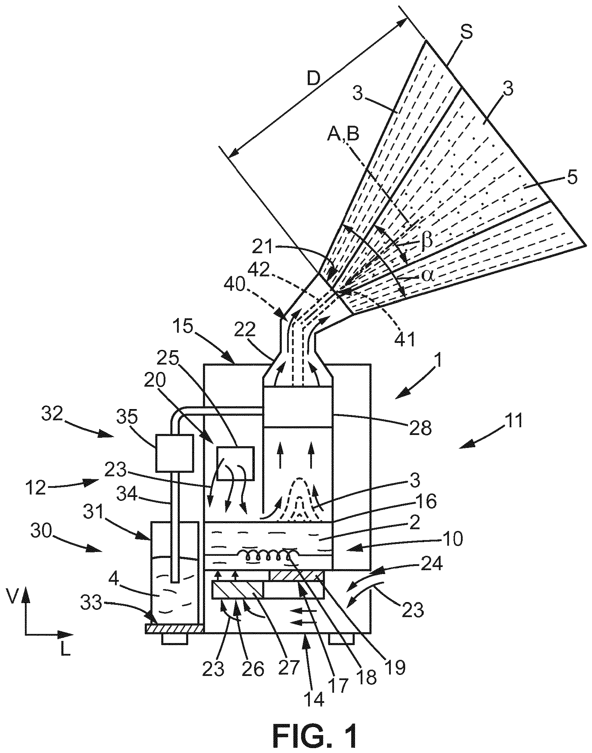

- la figure 1 est une représentation schématique d'une face latérale d'un dispositif de pulvérisation mis en œuvre dans une méthode de traitement cosmétique selon un premier mode de réalisation de l'invention, la méthode de traitement cosmétique prévoyant de vaporiser une composition comprenant de l'eau par un système de vaporisation et de nébuliser une composition comprenant au moins une molécule choisie parmi une molécule non volatile et une molécule instable à la température par un système de nébulisation, le système de vaporisation et le système de nébulisation délivrant des flux respectivement selon des directions de sortie de vaporisation et de sortie de nébulisation parallèles et écartés l'une de l'autre selon une direction transversale,
- la figure 2 est une représentation schématique d'une face avant du dispositif de pulvérisation de la figure 1,
- la figure 3 est une représentation schématique d'une face latérale d'un dispositif de pulvérisation mis en œuvre dans une méthode de traitement cosmétique selon un deuxième mode de réalisation de l'invention, le système de vaporisation et le système de nébulisation délivrant des flux de manière coaxiale, respectivement selon des directions de sortie de vaporisation et de sortie de nébulisation sensiblement confondues,
- la figure 4 est une représentation en perspective d'un exemple de dispositif de pulvérisation selon le deuxième mode de réalisation de l'invention,
- la figure 5 est une représentation en élévation du dispositif de pulvérisation de la figure 4,
- la figure 6 est une représentation de l'organe de distribution de composition vaporisée du système de vaporisation et de l'organe de distribution de composition nébulisée du système de nébulisation du dispositif de pulvérisation de la figure 4,
- la figure 7 est une représentation en coupe selon l'orientation référencée VII-VII sur la figure 6 de l'organe de distribution de composition vaporisée et de l'organe de distribution de composition nébulisée,
- la figure 8 est une représentation en coupe selon l'orientation référencée VIII-VIII sur la figure 6 de l'organe de distribution de composition vaporisée et de l'organe de distribution de composition nébulisée,
- la figure 9 est une représentation agrandie du détail référencé IX sur la figure 8 illustrant l'un des trous par lequel l'organe de distribution de composition vaporisée délivre un flux de vapeur.

### Dispositif de pulvérisation

Sur les figures, le dispositif de pulvérisation 1 est adapté d'une part pour vaporiser une composition comprenant de l'eau 2 et d'autre part pour nébuliser une composition comprenant au moins une molécule non volatile et/ou instable à la température 4.

Il est ainsi possible de véhiculer à la fois un flux de vapeur 3 de composition comprenant de l'eau 2 et un flux de composition nébulisée 5 comprenant des gouttelettes de composition comprenant au moins une molécule non volatile et/ou instable à la température 4, sans dégrader cette dernière composition 4.

Pour ce faire, le dispositif de pulvérisation 1 comprend :
i. un système de vaporisation 10 adapté pour vaporiser la composition comprenant de l'eau 2 selon une direction de sortie de vaporisation A, et
ii. un système de nébulisation 30 adapté pour nébuliser la composition comprenant au moins une molécule choisie parmi une molécule non volatile et une molécule instable à la température 4 selon une direction de sortie de nébulisation B.

Dans les modes de réalisation représentés, les systèmes de vaporisation 10 et de nébulisation 30 sont distincts l'un de l'autre.

Comme il ressortira de la suite de la description, le dispositif de pulvérisation 1 peut également comprendre un organe d'activation permettant d'activer simultanément ou alternativement le système de vaporisation 10 et le système de nébulisation 30. Un tel organe d'activation peut comprendre un ou plusieurs circuits électriques et/ou électroniques. Il peut être commandé par une unité de commande comprenant un programmateur et/ou un dispositif logiciel permettant de définir un ou une pluralité de cycle(s) de traitement.

Le dispositif de pulvérisation 1 présente :
- des faces avant 11 et arrière 12 opposées et écartées l'une de l'autre selon une direction longitudinale L,
- des faces latérales 13 opposées et écartées l'une de l'autre selon une direction transversale T perpendiculaire à la direction longitudinale L,
- une base 14 et une partie supérieure 15 opposées et écartées l'une de l'autre selon une direction verticale V perpendiculaire aux directions longitudinale L et transversale T.

Le système de vaporisation 10 comprend une cuve 16 pour recevoir la composition comprenant de l'eau 2. La cuve 16 est, par exemple, disposée au voisinage de la base 14 du dispositif de pulvérisation 1. La cuve 16 est, de préférence, amovible par exemple sous la forme d'un réservoir rechargeable présentant une contenance adaptée pour une mise en œuvre d'une ou plusieurs étapes de vaporisation de la composition comprenant de l'eau 2. En variante, la cuve 16 pourrait être une partie fixe du système de vaporisation 10 dans laquelle la composition comprenant de l'eau 2 est versée.

Le système de vaporisation 10 comprend également un dispositif de chauffage 17 adapté pour vaporiser la composition de manière à former un flux de vapeur 3. Le dispositif de chauffage 17 peut notamment comprendre une ou plusieurs résistances électriques 18 disposées dans ou au contact de la cuve 16, alors en matériau thermiquement conducteur, pour former le flux de vapeur 3. La formation du flux de vapeur 3 peut être favorisée par une formation préalable de gouttelettes grâce notamment à un élément d'excitation 19 par ultra-sons disposé en contact avec un fond de la cuve 16.

Le système de vaporisation 10 comprend un organe de distribution de composition vaporisée 20 adapté pour orienter et délivrer le flux de vapeur 3 au travers d'une ouverture de vaporisation 21, disposée dans la partie supérieure 15 du dispositif de pulvérisation 1, selon la direction de sortie de vaporisation A. Sur les figures, l'organe de distribution de composition vaporisée 20 comprend un conduit 22 dont une extrémité inférieure est placée dans la cuve 16, au-dessus d'un niveau maximum de composition contenant de l'eau 2, sensiblement en regard de l'élément d'excitation 19 du dispositif de chauffage 17. Le conduit 22 présente une extrémité supérieure conformée de toute manière appropriée pour diriger et faire converger le flux de vapeur 3 de la composition comprenant de l'eau 2 vers l'ouverture de vaporisation 21.

Afin d'entrainer le flux de vapeur 3, un flux d'air 23 peut être généré entre un orifice d'admission 24 ménagé dans la base 14 du dispositif de pulvérisation 1 et un orifice d'évacuation 25 débouchant au-dessus de la cuve 16 en amont de l'extrémité inférieure du conduit 22. L'organe de distribution de composition vaporisée 20 peut alors comprendre un élément d'aspiration 26 disposé dans la base 14. Le flux d'air 23 peut être chauffé par tout élément de chauffage 27 approprié, par exemple disposé en amont de l'orifice d'évacuation 25. Un élément de chauffage additionnel 28 peut être prévu dans le conduit 22, en amont de l'ouverture de vaporisation 21. Selon un mode de réalisation, la température du flux de vapeur 3 au niveau de l'ouverture de vaporisation 21 est comprise entre 90°C et 110°C, de préférence d'environ 100°C.

La composition comprenant au moins une molécule non volatile et/ou instable à la température 4 est, par exemple, approvisionnée dans un flacon 31, formant un réservoir, présentant une contenance adaptée pour une mise en œuvre d'une étape de nébulisation de la composition comprenant au moins une molécule non volatile et/ou une molécule instable à la température 4. Le flacon 31 peut être interchangeable de manière à être facilement mis en place ou retiré du système de nébulisation 30. Il est ainsi possible de changer facilement de composition comprenant au moins une molécule non volatile et/ou instable à la température 4 en choisissant un flacon 31 parmi une gamme de flacons 31 comprenant respectivement des compositions différentes. En variante, le système de nébulisation 30 pourrait comprendre plusieurs flacons 31 distincts formant réservoir. Le système de nébulisation est alors adapté pour prélever une composition comprenant au moins une molécule non volatile et/ou instable à la température 4 comprise dans l'un des flacons 31 en fonction du choix des molécules actives à nébuliser. Selon une autre variante, le réservoir contenant la composition comprenant au moins une molécule non volatile et/ou instable à la température 4 peut avoir une contenance adaptée pour une mise en œuvre de plusieurs étapes de nébulisation. Il pourrait, par ailleurs, être fixe, la composition comprenant au moins une molécule non volatile et/ou instable à la température 4 y étant versée de toute manière appropriée.

Le système de nébulisation 30 comprend un organe d'extraction 32 adapté pour extraire du réservoir 31 la composition comprenant au moins une molécule non volatile et/ou instable à la température 4. En particulier, l'organe d'extraction 32 est adapté pour être mis en communication de fluide avec le flacon 31 lorsque celui-ci est monté sur l'organe d'extraction 32. Sur les figures, l'organe d'extraction 32 peut comprendre un siège 33 sur lequel le flacon 31 est monté de manière amovible et un tube 34 prévu afin de prélever la composition comprenant au moins une molécule non volatile et/ou instable à la température 4 dans le flacon 31. Le tube 34 peut être relié à une pompe 35 afin de faciliter l'extraction de la composition comprenant au moins une molécule non volatile et/ou instable à la température 4.

La composition comprenant au moins une molécule non volatile et/ou instable à la température 4 est ensuite acheminée vers un dispositif de nébulisation 40 adapté pour nébuliser la composition comprenant au moins une molécule non volatile et/ou instable à la température 4 de manière à former le flux de composition nébulisée 5. Le dispositif de nébulisation 40 comprend un organe de distribution de composition nébulisée 42 adapté pour orienter et délivrer le flux de composition nébulisée au travers d'une ouverture de nébulisation 41, selon la direction de sortie de nébulisation B. L'organe de distribution de composition nébulisée 42 comprend tout système d'extraction et de délivrance d'un nuage de gouttelettes avec une pression suffisante pour atteindre la surface de la peau considérée. Le dispositif de nébulisation 40 peut comprendre un ou plusieurs éléments piézo-électriques, comme par exemple une aiguille piézo-électrique ou une grille piézo-électrique, placés dans un conduit. En variante, le dispositif de nébulisation 40 pourrait comprendre une buse de pulvérisation. La nébulisation peut être également obtenue en mettant en œuvre un système à base d'air comprimé.

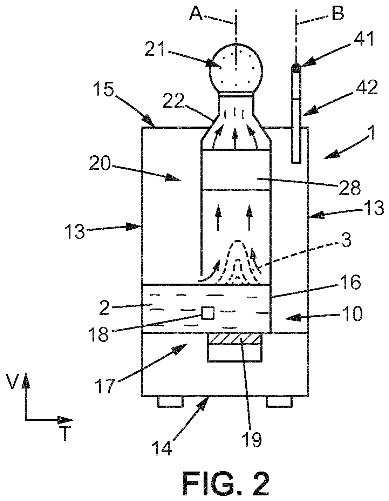

Dans le premier mode de réalisation représenté sur les figures 1 et 2, l'ouverture de nébulisation 41 est disposée dans la partie supérieure 15 du dispositif de pulvérisation 1 de telle manière que la direction de sortie de nébulisation B soit parallèle à la direction de sortie de vaporisation A. L'ouverture de nébulisation 41 est néanmoins décalée selon la direction transversale T par rapport à l'ouverture de vaporisation 21 de telle manière que :
- soit le flux de composition nébulisée 5 issu de l'ouverture de nébulisation 41 n'entre pas en contact avec le flux de vapeur 3 issu de l'ouverture de vaporisation 21,
- soit le flux de composition nébulisée 5 issu de l'ouverture de nébulisation 41 entre en contact avec le flux de vapeur 3 issu de l'ouverture de vaporisation 21 à une distance suffisante pour que la température du flux de vapeur 3 n'altère pas la molécule active contenue dans le flux de composition nébulisée 5.

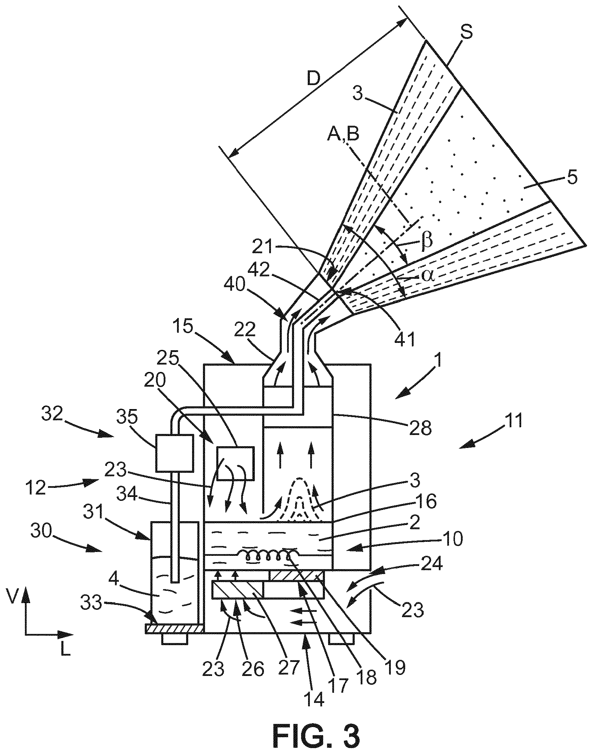

Dans un deuxième mode de réalisation représenté sur la figure 3, la direction de sortie de vaporisation A et la direction de sortie de nébulisation B sont sensiblement confondues. Le flux de vapeur 3 et le flux de composition nébulisée 5 sont alors coaxiaux. Ils peuvent notamment être concentriques. Selon un mode de réalisation, le flux de composition nébulisée 5 est entouré par le flux de vapeur 3.

Le dispositif de nébulisation 40, par exemple jusqu'à l'ouverture de nébulisation 41, peut comprendre des organes d'isolation thermique de manière à éviter que la composition comprenant au moins une molécule non volatile et/ou instable à la température 4 ne se dégrade.

Selon un mode de réalisation, la position de l'ouverture de vaporisation 21 du système de vaporisation 10 permettant la sortie du flux de vapeur 3 peut être variable et par exemple réglable en fonction de paramètres liés au type de soin que l'on désire programmer ; des moteurs peuvent par exemple être prévus et paramétrés à cet effet.

Selon un mode de réalisation, la position de l'ouverture de nébulisation 41 du système de nébulisation 30 permettant la sortie du flux de composition nébulisée 5 peut être variable et par exemple réglable en fonction de paramètres liés au type de soin que l'on désire programmer ; des moteurs peuvent par exemple être prévus et paramétrés à cet effet.

Le flux de vapeur 3 issu de l'ouverture de vaporisation 21 est de forme conique, par exemple un cône de révolution, dont l'axe correspond à la direction de sortie de vaporisation A. Le flux de composition nébulisée 5 issu de l'ouverture de nébulisation 41 est de forme conique, par exemple un cône de révolution, dont l'axe correspond à la direction de sortie de nébulisation B. A titre d'exemple, le flux de composition nébulisée 5 est distribué selon un cône ayant un angle d'ouverture α d'environ de 30° à 40° et le flux de vapeur 3 entoure le flux de composition nébulisée 5 à l'intérieur d'un cône ayant un angle d'ouverture β d'environ de 50° à 60°.

Le dispositif de pulvérisation 40 de molécules non volatiles et/ou instables à la température 4 est par exemple destiné à être placé à une distance de traitement D comprise entre environ 20 cm et 30 cm d'un visage et les flux qui en sont issus couvrent par exemple une surface S comprise entre environ 300 et 400 cm² dudit visage.

Selon un mode de réalisation, le flux de vapeur 3 de la composition comprenant de l'eau 2 arrivant au contact de la peau est formé de gouttelettes d'au moins 50 µm de diamètre et dont la température est comprise entre 35°C et 45°C, de préférence entre 36 °C et 40 °C.

Selon un mode de réalisation, le flux de composition nébulisée 5, comprenant au moins une molécule non volatile et/ou instable à la température, arrive au contact de la peau à une température comprise entre 15°C et 30°C, de préférence entre 20 °C et 25 °C.

### EXEMPLE

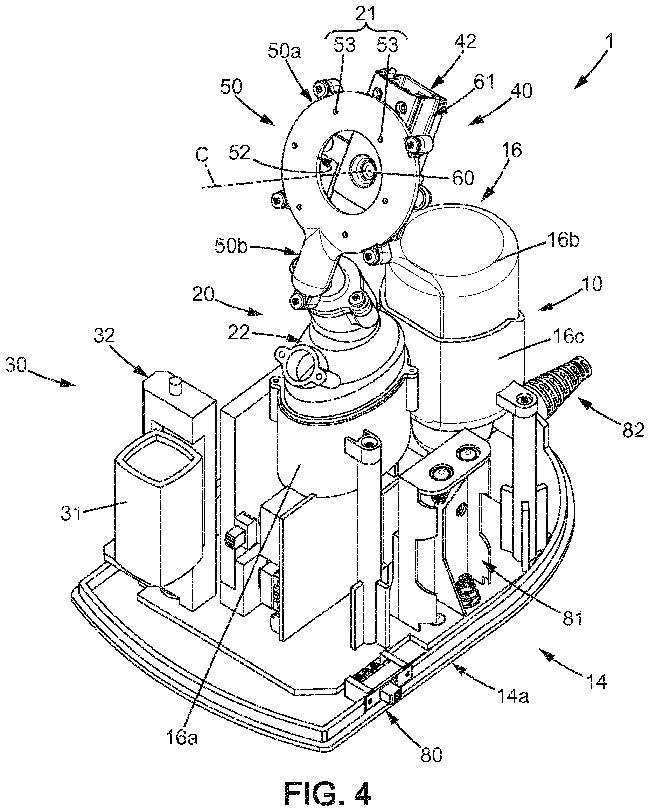

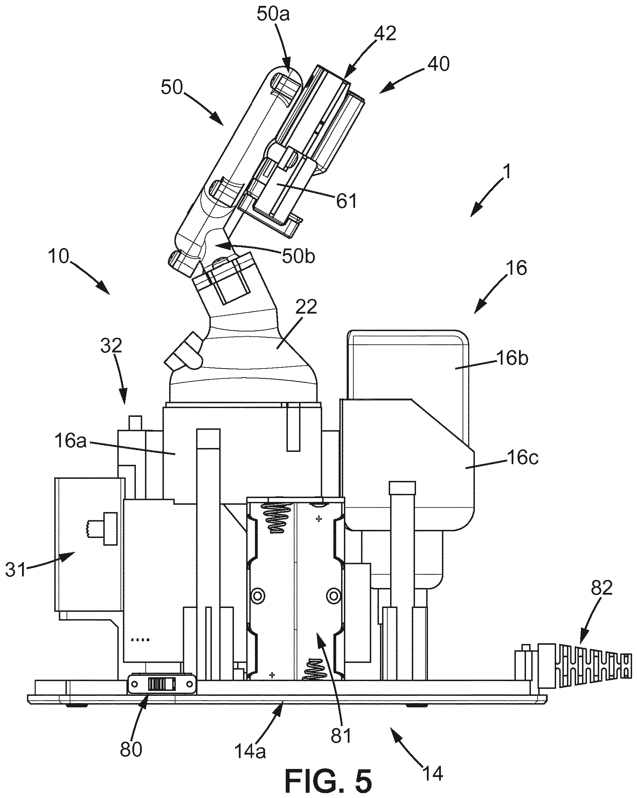

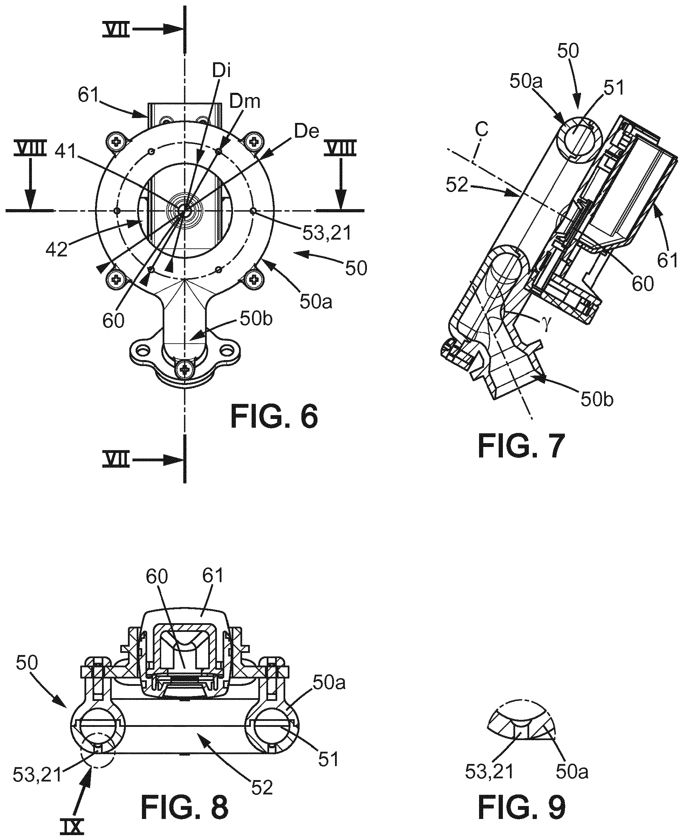

Les figures 4 à 9 illustrent un exemple d'un dispositif de pulvérisation 1 pouvant être mis en œuvre pour, comme décrit précédemment, vaporiser une composition comprenant de l'eau 2 et nébuliser une composition comprenant au moins une molécule non volatile et/ou instable à la température 4.

Conformément à la description qui en a été faite, le dispositif de pulvérisation 1 comprend le système de vaporisation 10 et le système de nébulisation 30 distincts l'un de l'autre et montés sur un socle 14a de la base 14.

La cuve 16 du système de vaporisation 10 comporte une chambre de vaporisation 16a avec laquelle le dispositif de chauffage 17 coopère pour générer le flux de vapeur 3 et dans laquelle le flux d'air peut circuler. La cuve 16 comprend également une cartouche 16b interchangeable alimentant la chambre de vaporisation 16a en composition comprenant de l'eau 2. La cartouche 16b peut être emmanchée dans un logement 16c adapté pour mettre en communication de fluide la cartouche 16b et la chambre de vaporisation 16a. L'extrémité inférieure du conduit 22 de l'organe de distribution de composition vaporisée 20 est placé au-dessus de la chambre de vaporisation 16a pour orienter et délivrer le flux de vapeur 3 au travers de l'ouverture de vaporisation 21.

L'organe de distribution de composition vaporisée 20 comprend une buse de vaporisation 50 solidarisée à une extrémité supérieure du conduit 22. En particulier, la buse 50 comporte une partie annulaire 50a autour d'un axe central C. La partie annulaire 50a de la buse de vaporisation 50, par exemple de section circulaire et présentant un contour circulaire, comporte intérieurement une chambre 51 et délimite une ouverture centrale 52. La partie annulaire 50a de la buse de vaporisation 50 comporte plusieurs trous 53, par exemple 6 sur les figures, mettant en communication la chambre 51 et l'extérieur. Les trous 53 sont équirépartis sur une face avant de la partie annulaire 50a de la buse de vaporisation 50 destinée à être orientée vers le visage de la consommatrice. Ils sont axiaux et agencés en correspondance avec un diamètre moyen Dm de la partie annulaire 50a de la buse de vaporisation 50. Les trous 51 de la buse de vaporisation 50 forment alors l'ouverture de vaporisation 21. En variante, la buse de vaporisation 50 et, en particulier, sa partie annulaire pourraient présenter tout autre contour et toute autre section appropriés et notamment polygonal, elliptique ou autre. En outre, tout autre nombre et tout autre agencement de trous 53 pourrait être prévu. En particulier, les trous 53 pourraient être inclinés par rapport à l'axe central C et décalés vers l'intérieur ou vers l'extérieur par rapport au diamètre moyen Dm.

La buse de vaporisation 50 comprend également un raccord 50b permettant de raccorder, le cas échéant de manière amovible, et de mettre en communication de fluide la chambre 51 et le conduit 22. Le raccord 50b est, de préférence, incliné d'un angle γ par rapport à un plan de la partie annulaire 50a pour pouvoir délivrer le flux de vapeur 3 vers l'avant et vers le haut par rapport à un conduit 22 sensiblement vertical.

A titre d'exemple non limitatif, la partie annulaire 50a peut présenter un diamètre intérieur Di correspondant à l'ouverture centrale 52 de 28 mm et un diamètre extérieur De de 52 mm, le diamètre moyen Dm étant de 39,60 mm. L'angle y entre le raccord 50b et le plan de la partie annulaire 50a est de 125°. Les trous présentent un diamètre de 1,7 mm.

Un flux de vapeur 3 généré par le dispositif de chauffage 17 développant une puissance comprise entre 250 W et 350 W alimente la chambre 51 de la partie annulaire 50a de la buse de vaporisation par l'intermédiaire du raccord 50b et peut être délivré vers l'extérieur au travers des trous 53 avec la distribution de température suivante :
- à 10 cm de l'organe de distribution de composition vaporisée 20 : entre 38,0°C et 42,0°C,
- à 15 cm de l'organe de distribution de composition vaporisée 20 : entre 35,0°C et 39°C,
- à 20 cm de l'organe de distribution de composition vaporisée 20 : entre 34,0°C et 36,0°C.

Un flacon 31 choisi parmi une gamme de flacons 31 est monté de manière amovible sur l'organe d'extraction 32 du système de nébulisation 30 de manière à alimenter l'organe de distribution de composition nébulisée 42 du dispositif de nébulisation 40 en composition comprenant au moins une molécule non volatile et/ou instable à la température 4.

L'organe de distribution de composition nébulisée 42 se présente sous la forme d'une grille piézoélectrique 60 comportant un ou plusieurs orifices formant l'ouverture de nébulisation 41. Les orifices présentent, par exemple, un diamètre compris entre 7 µm et 15 µm. Sur les figures, sans y être limitée, la grille piézoélectrique 60 est montée sur un boîtier 61 logeant des éléments d'actionnement de la grille piézoélectrique 60. Le boîtier 61 est solidarisé à la partie annulaire 50a de la buse de vaporisation 50 de telle manière que la grille piézoélectrique 60 soit disposée au centre de l'ouverture centrale 52 de la partie annulaire. Le flux de composition nébulisée 5 peut ainsi être délivré selon l'axe central C de la partie annulaire 50a de la buse de vaporisation 50. Conformément au deuxième mode de réalisation décrit précédent, l'organe de distribution de composition vaporisée 20 et l'organe de distribution de composition nébulisée 42 sont alors conformés et agencés de telle manière que les directions de sortie de vaporisation A et de nébulisation B sont confondues.

A titre d'exemple non limitatif, la grille piézoélectrique 60 peut être choisie parmi :
- une grille piézoélectrique réalisée en alliage de nickel et de cobalt cobalt (Ni-Co) et présentant des orifices de 7 µm de diamètre, produisant un débit de 0,54 ml/min,
- une grille piézoélectrique réalisée en acier inoxydable et présentant des orifices de 12 µm de diamètre, produisant un débit de 0,52 ml/min,
- une grille piézoélectrique réalisée en acier inoxydable et présentant des orifices de 15 µm de diamètre, produisant un débit entre 1,5 ml/min et 1,6 ml/min.

L'ensemble des composants peut être logé à l'intérieur d'un carter, non représenté, solidarisé au socle 14 et présentant des ouvertures agencées pour permettre le passage des flux de vapeur 3 et de composition nébulisée 5 ainsi que le changement de cartouche 16b et de flacon 31.

Le dispositif de pulvérisation 1 peut être activé par l'intermédiaire d'un organe d'activation 80 permettant d'alimenter les composants électriques et électroniques des systèmes de vaporisation 10 et nébulisation 30 en énergie provenant d'une source d'énergie autonome, telle qu'une ou plusieurs piles ou batteries placées dans un logement adapté 81, ou d'une source d'énergie reliée à un réseau éléctrique. La distribution des flux de vapeur 3 et de composition nébulisée 5 peut ensuite être réalisée de toute manière appropriée, le cas échéant, par l'intermédiaire d'une programmation enregistrée dans l'unité de commande.

### Molécules non volatiles et/ou instables à la température

Le dispositif de pulvérisation 1 permet la nébulisation de molécules non volatiles et/ou instables à la température.

Une méthode de pulvérisation mettant en œuvre le dispositif de pulvérisation précédemment décrit prévoit de vaporiser la composition comprenant de l'eau 2 grâce au système de vaporisation 10 et de nébuliser simultanément ou alternativement la composition comprenant au moins une molécule non volatile et/ou instable à la température 4.

On entend par « molécule non volatile » au sens de la présente demande, une molécule qui, une fois solubilisée dans l'eau ou dans un solvant approprié, ne s'évapore pas à une température comprise entre la température ambiante (25°C) et 130°C, à pression atmosphérique.

En particulier, lorsqu'elle est laissée à température ambiante, une molécule considérée comme non volatile au sens de la présente demande ne s'évapore pas pendant au moins une heure, et de préférence pendant au moins deux heures.

Lorsqu'elle est chauffée à température comprise entre 70 et 130°C, une molécule considérée comme non volatile au sens de la présente demande ne s'évapore pas pendant une durée comprise entre 5 et 120 minutes.

On entend par « molécules instables à la température » au sens de la présente demande, une molécule dont la structure chimique est modifiée lorsqu'elle est chauffée à température supérieure ou égale à 70°C, par exemple comprise entre 70 et 130°C, pendant une durée comprise entre 1 seconde et cinq minutes.

Les molécules véhiculées par le dispositif de pulvérisation sont de préférence non entrainables à la vapeur, c'est-à-dire qu'une fois solubilisée dans l'eau ou dans un solvant approprié, elles ne sont pas emportées par un courant de valeur d'eau obtenu à une température comprise entre 80 et 120°C pendant une durée allant de 1 minute à 1 heure.

Les molécules mises en œuvre dans le cadre de la présente demande sont cosmétiquement actives ou acceptables, c'est-à-dire qu'une composition les contenant confère un effet cosmétique notamment sur les matières kératiniques telles que la peau, par rapport à une même composition ne les contenant pas.

Les molécules susceptibles d'être véhiculées au moyen du dispositif selon l'invention présentent de préférence une densité théorique (mesurée à 100°C et à pression atmosphérique) supérieure à la densité de l'eau lors de son passage en phase vapeur à une température de 100°C, c'est à dire supérieure à 0.9584.

On entend par « densité théorique » le rapport de la masse d'un composé sur son volume à une température donnée ou issue d'une table de données connue d'un homme du métier. Les molécules susceptibles d'être véhiculées au moyen du dispositif de pulvérisation 1 présentent de préférence une masse moléculaire comprise entre 50 et 2000 g/mol.

A titre d'exemple de molécules susceptibles d'être véhiculées au moyen du dispositif de pulvérisation 1, on peut citer :
- les flavan-3-ols,
- les vitamines des groupes B (en particulier B1, B3, B6, B9),
- la vitamine C ou acide ascorbique et ses dérivés de type glucoside ou esterifaint un acide aminé de type lysine ou proline,
- la vitamine E ou tocopherol, ou ses dérivés en particulier phosphate ou acétate,
- les tocotrienols,
- les alcaloïdes de type xanthine, méthyl-, diméthyl- ou triméthyl-xanthine,
- les acides aminés instables à la température, en particulier la lysine, la théanine, la proline, le tryptophane,
- les lipides ou acides gras possédant au moins une double liaison insaturée
- les stérols de type cholestérol ou phytosterols et leurs dérivés
- les oligopeptides (jusqu'au hexapeptide)
- les glycols de type glycérol ou propylène glycol,
- les polyholosides de type acides hyaluronique de masse moléculaire comprise entre 50 et 2000 g/mol,
- les alpha et beta hydyroxyacides, tels que l'acide malique, l'acide citrique ou l'acide salicylique,
et leurs mélanges.

Selon un mode préféré de réalisation, les molécules mises en œuvre dans le cadre de la présente invention sont des flavan-3-ols (aussi appelés flavanols ou catéchines), sous famille de flavonoïdes.

Les flavan-3-ols sont caractérisés dans la classe des flavanoïdes par leur hétérocycle central C ne comportant qu'une seule substitution en 3 par un hydroxyle OH :

| | |
|---|---|
| 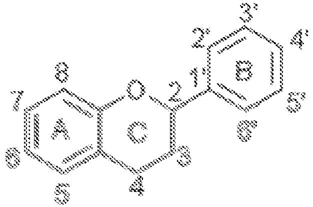 | 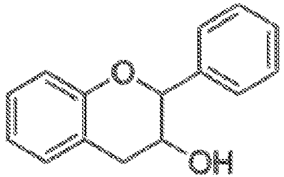 |
| Squelette numéroté des flavonoïdes | Squelette de base des flavan-3-ol |

Ils se déclinent en une série de composés suivant les substitutions par des hydroxyles phénoliques sur les cycles A et B. Ces hydroxyles peuvent être libres ou éthérifiés ou être engagés dans des liaisons hétérosides.

Selon un mode préféré de réalisation, le flavan-3-ol est la catéchine ou catéchol ou ses dérivés qui comportent des hydroxyles OH en 5 et 7 sur le cycle A et en 4' et 5' sur le cycle B. La catéchine existe sous forme de plusieurs stéréo-isomères provenant de ses deux carbones asymétriques et peut donc être l'un des composés suivants :

| | |
|---|---|
| (+)-catéchine | 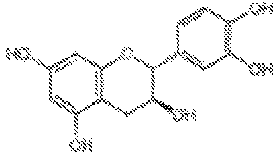 |
| (-)-épicatéchine | 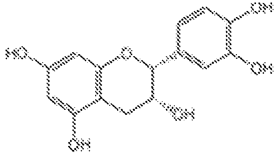 |
| (-)-catéchine | 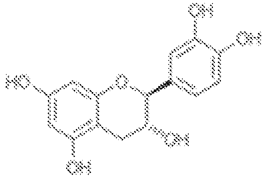 |
| (+)-épicatéchine | 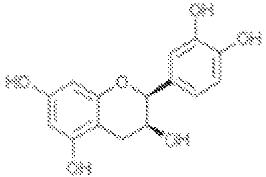 |

Alternativement, le groupement hydroxyle porté par la catéchine en position 3 peut être éthérifié par un alkyle en C1 à C30, et notamment un méthyl, éthyle, propyle ou galloyle. De telles molécules sont par exemple l'épicatéchine gallate de structure :

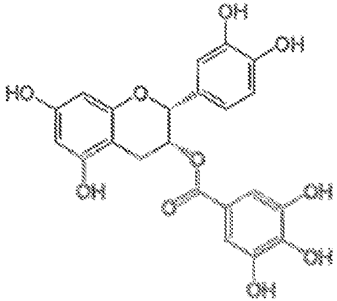

Selon un autre mode préféré de réalisation, le flavan-3-ol comporte des groupements hydroxyles OH en 5 et 7 sur le cycle A et en 3' 4' et 5' sur le cycle B comme par exemple l'épigallocatéchine gallate de structure :

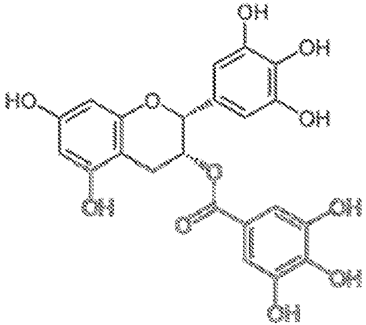

Les molécules susceptibles d'être véhiculées au moyen du dispositif de pulvérisation 1 peuvent, de préférence, être des procyanidines (ou procyanidols), c'est-à-dire des molécules constituées par une ou plusieurs unités flavan-3-ol précédemment décrites.

En particulier, les procyanidines peuvent être des dimères ou des trimères constitués à partir de deux ou trois unités catéchines liées entre elles. Les dimères les plus communs sont :
les procyanidines de type B-1, B-2, B-3 et B-4 : dimères constitués à partir des deux unités (+)-catéchol et (-)-épicatéchol, liées en C-4 → C-8 : 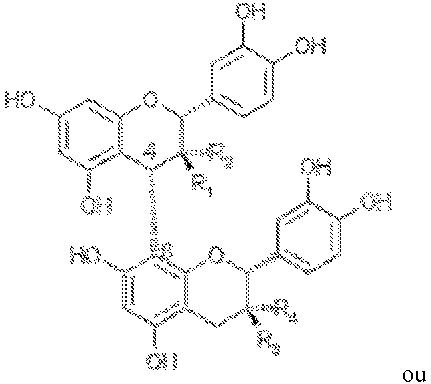
- les procyanidines type B-5, B-6, B-7 et B-8 : dimères constitués aussi à partir des deux unités (+)-catéchol et (-)-épicatéchol mais avec une liaison en C-4 → C-6.

Les flavan-3-ols se trouvent notamment dans les plantes telles que le théier (et notamment les feuilles de thé), et dans les fruits comme le raisin (en particulier les pépins de raisin), la fève de cacao, la mûre, la cerise, l'abricot, la framboise, la peau des pommes, la prune ou la fraise.

### Composition comprenant au moins une molécule non volatile et/ou instable à la température 4

La composition 4 mise en œuvre dans le dispositif de pulvérisation 1, comprenant au moins une molécule non volatile et/ou instable à la température, peut se présenter sous toute forme galénique permettant sa nébulisation, et notamment sous la forme d'une solution, d'une suspension, d'une émulsion fine type microémulsion ou nanoémulsion, d'une lotion, d'un sérum.

Dans le cas d'une émulsion, il peut s'agir d'une émulsion eau-dans-huile ou huile-dans-eau, de préférence huile-dans-eau.

De préférence, la composition 4 présente une viscosité comprise entre 0,1 et 500 mPa.s, de préférence 1 à 100 mPa.s, et plus préférentiellement entre 1 et 50 mPa.s.

La viscosité de la composition est mesurée par un rhéomètre de chez TA instruments de type ARES G2 avec un système de cylindres coaxiaux de type Couette, pendant une durée de cinq minutes.

Les molécules non volatiles et/ou instables à la température sont de préférence introduites dans la composition destinée à être nébulisée selon leur dose d'emploi en cosmétique correspondant à leur dose d'activité biologique ou cosmétique, soit en une teneur allant généralement de 0.001 à 5% en poids, par rapport au poids total de la composition, de préférence, de 0.01 à 1%.

La composition 4 mise en œuvre dans le dispositif de pulvérisation 1 peut également comprendre tout autre constituant classiquement mis en œuvre dans les compositions cosmétiques, comme de l'eau, un solvant organique miscible à l'eau, des huiles, des tensioactifs, des parfums, des conservateurs, des neutralisants, des filtres solaires, des hydratants, des composés auto-bronzants, les sequestrants, les agents filmogènes, et leurs mélanges.

Outre les molécules non volatiles et/ou instables à la température cosmétiquement actives, la composition nébulisée 5 dans le dispositif de pulvérisation peut par ailleurs contenir un ou plusieurs actifs hydrophiles ou lipophiles, qui peuvent être choisis parmi les antioxydants, les agents hydratants, les émollients, les agents anti-vieillissement, les agents anti-pollution, les agents kératolytiques, les astringents, les anti-inflammatoires non stéroïdiens, les agents blanchissants, et les agents anti-âge.

En particulier, les agents anti-âge peuvent notamment être choisi parmi : les agents stimulant l'expression de la tensine 1; les agents stimulant l'expression de la fructosamine-3-kinase ou de sa protéine apparentée (FN3K RP); les agents stimulant la production de facteurs de croissance; les agents anti-glycation ou déglycants; les agents augmentant la synthèse de collagène ou prévenant sa dégradation (agents anti-collagénases, notamment inhibiteurs de métalloprotéinases matricielles); les agents augmentant la synthèse d'élastine ou prévenant sa dégradation (agents anti-élastases); les agents augmentant la synthèse de glycosaminoglycanes ou de protéoglycanes ou prévenant leur dégradation (agents anti-protéoglycanases); les agents stimulant la synthèse d'intégrines par les fibroblastes ; les agents augmentant la prolifération ou la différenciation des kératinocytes; les agents augmentant la prolifération des fibloblastes; les agents anti-oxydants ou anti-radicalaires ou anti-pollution; et leurs mélanges, sans que cette liste ne soit limitative.

Les différents constituants de la composition nébulisée par le dispositif selon l'invention devraient, de préférence, être stables à des températures comprises entre 50 et 100°C, de préférence entre 60 et 80°C.

### Composition 2 destinée à former une vapeur d'eau 3

La composition 2 mise en œuvre dans le système de vaporisation 10 du dispositif de pulvérisation 1, comprend au moins de l'eau.

Elle peut également comprendre tout constituant classiquement mis en œuvre dans les compositions cosmétiques tel que décrit précédemment, sous réserve qu'il soit stable à des températures comprises entre 90 et 130°C, de préférence entre 100 et 110°C.

La composition 2 mise en œuvre dans le système de vaporisation 10 du dispositif de pulvérisation 1, peut également comprendre tout actif hydrophile ou lipophile volatile (par exemple des huiles essentielles ou des parfums) ou stable à la température.

### Traitement cosmétique

Une méthode de traitement cosmétique de la peau comprenant la pulvérisation sur la peau de molécules non volatiles et/ou instables à la température au moyen du dispositif de pulvérisation est maintenant décrite.

Une telle méthode comprend notamment une étape de pulvérisation simultanée sur les matières kératiniques, et notamment la peau, d'un courant de vapeur d'eau 3 et d'une nébulisation d'une composition comprenant au moins une molécule non volatile et/ou instable à la température 4 au moyen du dispositif de pulvérisation 1 précédemment décrit, à une distance de traitement D comprise entre 20 cm et 30 cm de la peau pendant une durée comprise entre 1 et 10 minutes, de préférence entre 1 minute 30 secondes et 5 minutes.

Selon un mode de réalisation, la méthode de traitement cosmétique est mise en œuvre pour une application sur le visage d'une utilisatrice, en particulier sur une surface comprise entre 300 cm² et 400 cm² du visage.

Pour ce faire, l'utilisatrice choisit un flacon 31 contenant une composition comprenant au moins une molécule non volatile et/ou instable à la température 4 parmi la gamme de flacons 31 et le monte sur l'organe d'extraction 32 du système de nébulisation 30. Elle place ensuite son visage à une distance comprise entre 20 cm et 30 cm des ouvertures de vaporisation 21 et de nébulisation 41 des systèmes de vaporisation 10 et de nébulisation 30.

Elle active ensuite le dispositif de pulvérisation 1 qui réalise, de façon alternative ou simultanée, selon le traitement cosmétique souhaité, une ou plusieurs vaporisations de la composition comprenant de l'eau 2 et une ou plusieurs nébulisations de la composition comprenant au moins une molécule choisie parmi une molécule non volatile et une molécule instable à la température 4. Par exemple, le traitement cosmétique peut comprendre :
- une première étape de pulvérisation d'un courant de vapeur d'eau 3 uniquement au cours de laquelle seule la composition contenant de l'eau 2 est vaporisée sur le visage de l'utilisatrice par le système de vaporisation 10,
- une deuxième étape de pulvérisation simultanée d'un courant de vapeur d'eau 3 et de la composition comprenant au moins une molécule non volatile et/ou instable à la température 4 par les systèmes de vaporisation 10 et de nébulisation 30 fonctionnant conjointement, et
- une troisième étape d'arrêt de la pulvérisation d'un courant de vapeur d'eau 3, pour ne pulvériser qu'une nébulisation de la composition comprenant au moins une molécule non volatile et/ou instable à la température 4.

Le contrôle des étapes de la méthode de traitement selon l'invention peut être opéré au moyen de tout système de régulation communément connu de l'homme du métier. La durée des étapes, leur ordre ou leur puissance peuvent être ajustés par l'utilisatrice en fonction de l'activité recherchée.

En particulier, l'ordre des étapes et leur durée peut être contrôlé au moyen d'une application, par exemple depuis un smartphone, pour être personnalisée aux besoins de chaque utilisatrice.

La présente invention est illustrée plus en détails dans les exemples non limitatifs suivants.

### EXEMPLES

### Exemple 1 : identification des molécules non volatiles et/ou instables à la température selon l'invention

### 1^{ère} expérience

5g de feuilles de thé (Camélia sinensis) ont été infusées pendant 4 minutes dans 50 mL d'eau purifiée portée à 86°C. La vapeur émise est récupérée pendant 6 minutes. Le volume collecté est d'environ 0.5 mL.

On a analysé la composition de la vapeur récupérée et du liquide après infusion des feuilles (dilution au 1/10°) pour y rechercher les molécules suivantes présentes dans les feuilles de thé ayant servi à l'expérience :
- la caféine
- la catéchine
- l'épicatéchine
- l'épigallocatéchine Gallate
- l'épicatéchine Gallate

Les 5 molécules d'intérêt ont été détectées par HPLC dans le liquide après infusion des feuilles.

Toutefois, seule la caféine a été détectée dans la vapeur émise pendant l'infusion des feuilles.

Les molécules de la famille des catéchines ne sont pas véhiculées par la vapeur.

### 2^{ème} expérience :

6g de feuille de thé ont été broyés pendant 2 fois 10s.

5g de la poudre grossière obtenue a été dispersée dans 250ml d'eau purifiée dans un ballon à fond rond de 500ml, puis le ballon a été placé dans un bain d'huile à température ambiante avec une consigne de chauffe à 120°C.

Le temps nécessaire à obtenir 120°C dans le bain d'huile est d'environ 30 minutes L'ébullition à l'intérieur du ballon a été obtenue 50 minutes après le début de l'expérience, sous agitation à 300rpm.

Après 65 minutes, la première goutte de distillation est récupérée.

Le produit de la distillation a été récupéré dans 10 vials HPLC. Les 10 vials ainsi que le surnageant des feuilles de thé dans l'eau (dilué au 1/10ème) ont été analysés en parallèle d'une gamme de témoin en Caféine, Catéchine, Epicatéchine, Epigallocatéchine Gallate (EGCG) et Epicatéchine Gallate, selon la méthode HPLC.

Après une heure de distillation, on ne retrouve aucun des composés recherchés dans la vapeur récupérée, en dehors de traces infimes d'EGCG.

On retrouve, en revanche, toutes les molécules d'intérêt dans le liquide après infusion des feuilles de thé. L'analyse du surnageant des feuilles de thé dans l'eau est indiquée dans le tableau ci-après:

| | Concentration en µg/ml |
|---|---|
| Caféine | 820 |
| Catéchine | 220 |
| Epicatéchine | 370 |
| Epigallocatéchine Gallate | 800 |
| Epicatéchine Gallate | 930 |

Ainsi, même après 1heure d'ébullition, les molécules de la famille des catéchines ne sont pas véhiculées par la vapeur.

### 3^{ème} expérience :

On a préparé une solution ayant la composition suivante :
- Extrait de feuilles de Camélia sinensis : 1g
- Hétérosides de Centella asiatica : 0.01g
- Acide ascorbique : 1g
- Glycerol : 0.5g
- Phenoxyethanol : 0.6 g
- Eau déminéralisée : QSP 100 ml

Cette solution a été introduite dans le flacon 10 du dispositif selon l'invention relié à un moyen de nébulisation de la composition. Une brume de vapeur chaude a été générée au moyen du dispositif de l'invention. Deux minutes après le début de la vaporisation de la vapeur chaude, la solution a été nébulisée en fines gouttelettes et diffusée sur la peau de l'utilisatrice conjointement à la vapeur pendant une durée de 1 minute et 30 s.

Ce traitement cosmétique a été renouvelé une fois par jour pendant 4 jours sur une peau propre et démaquillée.

Les caractéristiques de la peau sont évaluées par un expert 5 minutes après l'arrêt du traitement.

Le traitement a permis de protéger la peau de stress radicalaires, d'améliorer l'hydratation, l'éclat et les propriétés biomécaniques, notamment en terme de fermeté et/ou d'élasticité, supérieurs à ceux observés lors d'un même traitement mettant en œuvre la seule nébulisation de la solution sans brumisation conjointe d'une vapeur d'eau.

## Revendications

1. Méthode de traitement cosmétique de la peau comprenant une pulvérisation sur la peau d'au moins une molécule choisie parmi une molécule non volatile et une molécule instable à la température au moyen d'un dispositif de pulvérisation (1) comprenant :
i. un système de vaporisation (10) adapté pour vaporiser une composition comprenant de l'eau (2), et
ii. un système de nébulisation (30) adapté pour nébuliser une composition comprenant au moins une molécule choisie parmi une molécule non volatile et une molécule instable à la température (4),
la méthode de traitement cosmétique prévoyant de vaporiser la composition comprenant de l'eau (2) par le système de vaporisation (10) et de nébuliser la composition comprenant au moins une molécule choisie parmi une molécule non volatile et une molécule instable à la température (4) par le système de nébulisation (30).

2. Méthode de traitement cosmétique selon la revendication 1, prévoyant de vaporiser la composition comprenant de l'eau (2) et de nébuliser la composition comprenant au moins une molécule choisie parmi une molécule non volatile et une molécule instable à la température (4) simultanément.

3. Méthode de traitement cosmétique selon l'une quelconque des revendications 1 et 2, prévoyant de vaporiser la composition comprenant de l'eau (2) et de nébuliser la composition comprenant au moins une molécule choisie parmi une molécule non volatile et une molécule instable à la température (4) alternativement.

4. Méthode de traitement cosmétique selon les revendications 1 à 3, prévoyant de vaporiser la composition comprenant de l'eau (2) selon une direction de sortie de vaporisation (A) et de nébuliser la composition comprenant au moins une molécule choisie parmi une molécule non volatile et une molécule instable à la température (4) selon une direction de sortie de nébulisation (B), la direction de sortie de vaporisation (A) et la direction de sortie de nébulisation (B) étant sensiblement confondues.

5. Méthode de traitement cosmétique selon l'une quelconque des revendications 1 à 4, dans laquelle le système de vaporisation (10) comprend une cuve (16) pour recevoir la composition comprenant de l'eau (2), un dispositif de chauffage (17) adapté pour vaporiser ladite composition de manière à former un flux de vapeur (3), un organe de distribution de composition vaporisée (20) adapté pour orienter et délivrer ledit flux de vapeur (3) vers une ouverture de vaporisation (21).

6. Méthode de traitement cosmétique selon l'une quelconque des revendications 1 à 5, dans laquelle le système de nébulisation (30) comprend un réservoir (31) contenant la composition comprenant au moins une molécule choisie parmi une molécule non volatile et une molécule instable à la température (4), un organe d'extraction (32) adapté pour extraire la composition comprenant au moins une molécule choisie parmi une molécule non volatile et une molécule instable à la température (4) du réservoir (31), un dispositif de nébulisation (40) adapté pour nébuliser ladite composition de manière à former un flux de composition nébulisée (5), le dispositif de nébulisation (40) comprenant un organe de distribution de composition nébulisée (42) adapté pour orienter et délivrer ledit flux de composition nébulisée vers une ouverture de nébulisation (41).

7. Méthode de traitement cosmétique selon la revendication 6, dans laquelle le réservoir (31) est monté de manière amovible sur l'organe d'extraction (32), la méthode de traitement cosmétique prévoyant de choisir un réservoir (31) parmi une gamme de réservoirs (31) comprenant respectivement des compositions différentes, de monter le réservoir (31) choisi sur l'organe d'extraction (32), de nébuliser la composition contenue dans le réservoir (31) et de retirer le réservoir (31) de l'organe d'extraction (32).

8. Méthode de traitement cosmétique selon l'une quelconque des revendications 6 et 7, dans laquelle le dispositif de nébulisation (40) est comprend un composant choisi parmi une grille piézo-électrique et une buse de pulvérisation.

9. Méthode de traitement cosmétique selon l'une quelconque des revendications 1 à 8, dans laquelle la molécule choisie parmi une molécule non volatile et une molécule instable à la température présente une densité théorique, mesurée à 100°C et à pression atmosphérique, supérieure à 0.9584.

10. Méthode de traitement cosmétique selon l'une quelconque des revendications 1 à 9, dans laquelle la molécule choisie parmi une molécule non volatile et une molécule instable à la température présente une masse moléculaire comprise entre 50 et 2000 g/mol.

11. Méthode de traitement cosmétique selon l'une quelconque des revendications 1 à 10, dans laquelle la molécule choisie parmi une molécule non volatile et une molécule instable est choisie parmi :
- les flavan-3-ols,
- les vitamines des groupes B (en particulier B1, B3, B6, B9),
- la vitamine C ou acide ascorbique et ses dérivés de type glucoside ou esterifaint un acide aminé de type lysine ou proline,
- la vitamine E ou tocopherol, ou ses dérivés en particulier phosphate ou acétate,
- les tocotrienols,
- les alcaloïdes de type xanthine, méthyl-, diméthyl- ou triméthyl-xanthine,
- les acides aminés instables à la température, en particulier la lysine, la théanine, la proline, le tryptophane,
- les lipides ou acides gras possédant au moins une double liaison insaturée
- les stérols de type cholestérol ou phytosterols et leurs dérivés
- les oligopeptides (jusqu'au hexapeptide)
- les glycols de type glycérol ou propylène glycol,
- les polyholosides de type acides hyaluronique de masse moléculaire comprise entre 50 et 2000 g/mol,
- les alpha et beta hydyroxyacides, tels que l'acide malique, l'acide citrique ou l'acide salicylique,
et leurs mélanges.

12. Méthode de traitement cosmétique selon la revendication 11, dans laquelle la molécule choisie parmi une molécule non volatile et une molécule instable est un flavan-3-ol choisi parmi la catéchine, l'épicatéchine, l'épicatéchine gallate, l'épigallocatéchine gallate, et leurs mélanges.

13. Méthode de traitement cosmétique selon l'une quelconque des revendications 1 à 12, dans laquelle la composition comprenant au moins une molécule choisie parmi une molécule non volatile et une molécule instable à la température (4) présente une viscosité comprise entre 0,1 et 500 mPa.s, de préférence 1 à 100 mPa.s, et plus préférentiellement entre 1 et 50 mPa.s.

14. Méthode de traitement cosmétique selon l'une quelconque des revendications 1 à 13, dans laquelle la composition comprenant au moins une molécule choisie parmi une molécule non volatile et une molécule instable à la température comprend tout autre constituant mis en œuvre dans les compositions cosmétiques choisi parmi l'eau, un solvant organique miscible à l'eau, des huiles, des tensioactifs, des parfums, des conservateurs, des neutralisants, des filtres solaires, des hydratants, des composés auto-bronzants, les sequestrants, les agents filmogènes, et leurs mélanges.

15. Méthode de traitement cosmétique selon l'une quelconque des revendications 1 à 14, dans laquelle la composition comprenant au moins une molécule choisie parmi une molécule non volatile et une molécule instable à la température (4) comprend un ou plusieurs actifs hydrophiles ou lipophiles choisis parmi les antioxydants, les agents hydratants, les émollients, les agents anti-vieillissement, les agents anti-pollution, les agents kératolytiques, les astringents, les anti-inflammatoires non stéroïdiens, les agents blanchissants, et les agents anti-âge.

16. Méthode de traitement cosmétique selon l'une quelconque des revendications 1 à 15, prévoyant d'appliquer la composition comprenant de l'eau (2) et la composition comprenant au moins une molécule choisie parmi une molécule non volatile et une molécule instable à la température (4) sur le visage d'une utilisatrice.

17. Méthode de traitement cosmétique selon l'une quelconque des revendications 1 à 16, prévoyant de vaporiser la composition comprenant de l'eau (2) et de nébuliser la composition comprenant au moins une molécule choisie parmi une molécule non volatile et une molécule instable à la température (4) sur une surface (S) comprise entre 300 cm² et 400 cm² située à une distance de traitement (D) comprise entre 20 cm et 30 cm des systèmes de vaporisation (10) et de nébulisation (30).

18. Méthode de traitement cosmétique selon la revendication 17 lorsqu'elle dépend des revendications 5 et 6, dans laquelle le flux de vapeur (3) a une température comprise entre 90°C et 110°C au niveau de l'ouverture de vaporisation (21) et une température comprise entre 35°C et 45°C à la distance de traitement (D), et dans laquelle le flux de composition nébulisée (5) a une température comprise entre 15°C et 30°C à la distance de traitement (D).

## Patentansprüche

1. Verfahren zur kosmetischen Behandlung der Haut, umfassend ein Aufsprühen auf die Haut wenigstens eines Moleküls, ausgewählt aus einem nichtflüchtigen Molekül und einem temperaturinstabilen Molekül, mittels einer Sprühvorrichtung (1), umfassend:
i. ein Verdampfungssystem (10), welches zum Verdampfen einer wasserhaltigen Zusammensetzung (2) ausgebildet ist, und
ii. ein Vernebelungssystem (30), welches dazu ausgebildet ist, eine Zusammensetzung zu vernebeln, welche wenigstens ein Molekül, ausgewählt aus einem nichtflüchtigen Molekül und einem temperaturinstabilen Molekül (4), umfasst,
wobei das kosmetische Behandlungsverfahren das Verdampfen der wasserhaltigen Zusammensetzung (2) durch das Verdampfungssystem (10) und das Vernebeln der Zusammensetzung, welche wenigstens ein Molekül, ausgewählt aus einem nichtflüchtigen Molekül und einem temperaturinstabilen Molekül (4), umfasst, durch das Vernebelungssystem (30) aufweist.

2. Verfahren zur kosmetischen Behandlung nach Anspruch 1, umfassend das Verdampfen der wasserhaltigen Zusammensetzung (2) und das gleichzeitige Vernebeln der Zusammensetzung, welche wenigstens ein Molekül, ausgewählt aus einem nichtflüchtigen Molekül und einem temperaturinstabilen Molekül (4), umfasst.

3. Verfahren zur kosmetischen Behandlung nach einem der Ansprüche 1 und 2, umfassend das abwechselnde Verdampfen der wasserhaltigen Zusammensetzung (2) und Vernebeln der Zusammensetzung, welche wenigstens ein Molekül, ausgewählt aus einem nichtflüchtigen Molekül und einem temperaturinstabilen Molekül (4), umfasst.

4. Verfahren zur kosmetischen Behandlung nach einem der Ansprüche 1 bis 3, umfassend das Verdampfen der wasserhaltigen Zusammensetzung (2) in einer Verdampfungsauslassrichtung (A) und das Vernebeln der Zusammensetzung, welche wenigstens ein Molekül, ausgewählt aus einem nichtflüchtigen Molekül und einem temperaturinstabilen Molekül (4), umfasst, in einer Vemebelungsauslassrichtung (B), wobei die Verdampfungsauslassrichtung (A) und die Vernebelungsauslassrichtung (B) im Wesentlichen zusammenfallen.

5. Verfahren zur kosmetischen Behandlung nach einem der Ansprüche 1 bis 4, wobei das Verdampfungssystem (10) ein Gefäß (16) zur Aufnahme der wasserhaltigen Zusammensetzung (2), eine Heizvorrichtung (17), die dazu ausgebildet ist, die Zusammensetzung zu verdampfen, um einen Dampfstrom (3) zu bilden, und ein Verteilelement für die verdampfte Zusammensetzung (20) umfasst, das dazu ausgebildet ist, den Dampfstrom (3) zu einer Verdampfungsöffnung (21) zu leiten und zuzuführen.

6. Verfahren zur kosmetischen Behandlung nach einem der Ansprüche 1 bis 5, wobei das Vernebelungssystem (30) einen Tank (31) umfasst, der die Zusammensetzung enthält, welche wenigstens ein Molekül, ausgewählt aus einem nichtflüchtigen Molekül und einem temperaturinstabilen Molekül (4), umfasst, ein Extraktionselement (32), das dazu ausgebildet ist, die Zusammensetzung, welche wenigstens ein Molekül, ausgewählt aus einem nichtflüchtigen Molekül und einem temperaturinstabilen Molekül (4), umfasst, aus dem Tank (31) zu extrahieren, eine Vernebelungsvorrichtung (40), die dazu ausgebildet ist, die Zusammensetzung zu vernebeln, um einen Strom der vernebelten Zusammensetzung (5) zu bilden, wobei die Vernebelungsvorrichtung (40) ein Verteilelement für die vernebelte Zusammensetzung (42) umfasst, welches dazu ausgebildet ist, den Strom der vernebelten Zusammensetzung zu einer Vernebelungsöffnung (41) zu leiten und zuzuführen.

7. Verfahren zur kosmetischen Behandlung nach Anspruch 6, wobei der Tank (31) abnehmbar an dem Extraktionselelement (32) angebracht ist, wobei das Verfahren zur kosmetischen Behandlung das Auswählen eines Tanks (31) aus einer Reihe von Tanks (31), die jeweils unterschiedliche Zusammensetzungen umfassen, das Anbringen des ausgewählten Tanks (31) an dem Extraktionselement (32), das Vernebeln der in dem Tank (31) enthaltenen Zusammensetzung und das Entfernen des Tanks (31) von dem Extraktionselement (32) umfasst.

8. Verfahren zur kosmetischen Behandlung nach einem der Ansprüche 6 und 7, wobei die Vernebelungsvorrichtung (40) eine Komponente, ausgewählt aus einem piezoelektrischen Gitter und einer Sprühdüse, umfasst.

9. Verfahren zur kosmetischen Behandlung nach einem der Ansprüche 1 bis 8, wobei das Molekül, welches aus einem nichtflüchtigen Molekül und einem temperaturinstabilen Molekül ausgewählt ist, eine theoretische Dichte, gemessen bei 100°C und Atmosphärendruck, von mehr als 0,9584 aufweist.

10. Verfahren zur kosmetischen Behandlung nach einem der Ansprüche 1 bis 9, wobei das Molekül, welches aus einem nichtflüchtigen Molekül und einem temperaturinstabilen Molekül ausgewählt ist, ein Molekulargewicht zwischen 50 und 2000 g/mol aufweist.

11. Verfahren zur kosmetischen Behandlung nach einem der Ansprüche 1 bis 10, wobei das Molekül, ausgewählt aus einem nicht-flüchtigen Molekül und einem instabilen Molekül, ausgewählt ist aus:
- Flavan-3-olen,
- B-Vitaminen (insbesondere B1, B3, B6, B9),
- Vitamin C oder Ascorbinsäure und seinen Derivaten vom Glucosidtyp oder die eine Aminosäure vom Lysin- oder Prolin-Typ verestern,
- Vitamin E oder Tocopherol, oder seinen Derivaten, insbesondere Phosphat oder Acetat,
- Tocotrienolen,
- Alkaloiden vom Typ Xanthin, Methyl-, Dimethyl- oder Trimethyl-Xanthin,
- temperaturinstabilen Aminosäuren, insbesondere Lysin, Theanin, Prolin, Tryptophan,
- Lipiden oder Fettsäuren mit wenigstens einer ungesättigten Doppelbindung,
- Sterolen vom Typ Cholesterin oder Phytosterole und deren Derivaten,
- Oligopeptiden (bis zum Hexapeptid),
- Glykolen vom Typ Glycerin oder Propylenglykol,
- Polyholosiden vom Typ Hyaluronsäure mit einem Molekulargewicht zwischen 50 und 2000 g/mol,
- Alpha- und Beta-Hydroxysäuren, wie z. B. Äpfelsäure, Zitronensäure oder Salicylsäure,
und Mischungen davon.

12. Verfahren zur kosmetischen Behandlung nach Anspruch 11, wobei das Molekül, welches aus einem nichtflüchtigen Molekül und einem instabilen Molekül ausgewählt ist, ein Flavan-3-ol ist, ausgewählt aus Catechin, Epicatechin, Epicatechin-Gallat, Epigallocatechin-Gallat und Mischungen davon.

13. Verfahren zur kosmetischen Behandlung nach einem der Ansprüche 1 bis 12, wobei die Zusammensetzung, welche wenigstens ein Molekül, ausgewählt aus einem nicht flüchtigen Molekül und einem temperaturinstabilen Molekül (4), umfasst, eine Viskosität zwischen 0,1 und 500 mPa.s, bevorzugt zwischen 1 und 100 mPa.s und besonders bevorzugt zwischen 1 und 50 mPa.s aufweist.

14. Verfahren zur kosmetischen Behandlung nach einem der Ansprüche 1 bis 13, wobei die Zusammensetzung, welche wenigstens ein Molekül, ausgewählt aus einem nichtflüchtigen Molekül und einem temperaturinstabilen Molekül (4), umfasst, jede andere in kosmetischen Zusammensetzungen verwendete Komponente umfasst, ausgewählt aus Wasser, einem mit Wasser mischbaren organischen Lösungsmittel, Ölen, Tensiden, Duftstoffen, Konservierungsmitteln, Neutralisationsmitteln, Sonnenlichtfiltern, Feuchthaltemitteln, Selbstbräunungsverbindungen, Sequestriermitteln, Filmbildnern und Mischungen davon.

15. Verfahren zur kosmetischen Behandlung nach einem der Ansprüche 1 bis 14, wobei die Zusammensetzung, welche wenigstens ein Molekül, ausgewählt aus einem nichtflüchtigen Molekül und einem temperaturinstabilen Molekül (4), umfasst, einen oder mehrere hydrophile oder lipophile Wirkstoffe umfasst, ausgewählt aus Antioxidantien, Feuchthaltemitteln, Weichmachern, Anti-Aging-Mitteln, Anti-Verschmutzungsmitteln, keratolytischen Mitteln, Adstringenzien, nicht-steroidalen entzündungshemmenden Mitteln, Bleichmitteln und Anti-Aging-Mitteln.

16. Verfahren zur kosmetischen Behandlung nach einem der Ansprüche 1 bis 15, umfassend das Auftragen der wasserhaltigen Zusammensetzung (2) und der Zusammensetzung, welche wenigstens ein Molekül, ausgewählt aus einem nichtflüchtigen Molekül und einem temperaturinstabilen Molekül (4), umfasst, auf das Gesicht einer Anwenderin.

17. Verfahren zur kosmetischen Behandlung nach einem der Ansprüche 1 bis 16, umfassend das Verdampfen der wasserhaltigen Zusammensetzung (2) und das Vernebeln der Zusammensetzung, welche wenigstens ein Molekül, ausgewählt aus einem nichtflüchtigen Molekül und einem temperaturinstabilen Molekül (4), umfasst, auf einer Fläche (S) zwischen 300 cm² und 400 cm², die sich in einem Behandlungsabstand (D) zwischen 20 cm und 30 cm von dem Sprühsystem (10) und Vernebelungssystem (30) befindet.

18. Verfahren zur kosmetischen Behandlung nach Anspruch 17 in Abhängigkeit von den Ansprüchen 5 und 6, wobei der Dampfstrom (3) an der Dampföffnung (21) eine Temperatur zwischen 90°C und 110°C und in dem Behandlungsabstand (D) eine Temperatur zwischen 35°C und 45°C aufweist, und wobei der Strom der vernebelten Zusammensetzung (5) in dem Behandlungsabstand (D) eine Temperatur zwischen 15°C und 30°C aufweist.

## Claims

1. A method for cosmetic treatment of the skin comprising spraying onto the skin at least one molecule chosen from a non-volatile molecule and a temperature-unstable molecule by means of a spray device (1) comprising:
i. a vaporizing system (10) suitable for vaporizing a composition comprising water (2), and
ii. a nebulizing system (30) suitable for nebulizing a composition comprising at least one molecule chosen from a non-volatile molecule and a temperature-unstable molecule (4),
wherein the method for cosmetic treatment provides for vaporizing the composition comprising water (2) using the vaporizing system (10) and for nebulizing the composition comprising at least one molecule chosen from a non-volatile molecule and a temperature-unstable molecule (4) using the nebulizing system (30).

2. The method for cosmetic treatment as claimed in claim 1, providing for vaporizing the composition comprising water (2) and for nebulizing the composition comprising at least one molecule chosen from a non-volatile molecule and a temperature-unstable molecule (4) simultaneously.

3. The method for cosmetic treatment as claimed in either one of claims 1 and 2, providing for vaporizing the composition comprising water (2) and for nebulizing the composition comprising at least one molecule chosen from a non-volatile molecule and a temperature-unstable molecule (4) in an alternating manner.

4. The method for cosmetic treatment as claimed in claims 1 to 3, providing for vaporizing the composition comprising water (2) along a vaporization output direction (A) and for nebulizing the composition comprising at least one molecule chosen from a non-volatile molecule and a temperature-unstable molecule (4) along a nebulization output direction (B), the vaporization output direction (A) and the nebulization output direction (B) being substantially the same.

5. The method for cosmetic treatment as claimed in any one of claims 1 to 4, wherein the vaporizing system (10) comprises a tank (16) for receiving the composition comprising water (2), a heating device (17) suitable for vaporizing said composition so as to form a stream of vapor (3), a vaporized composition dispensing member (20) suitable for directing and delivering said stream of vapor (3) to a vaporizing aperture (21).

6. The method for cosmetic treatment as claimed in any one of claims 1 to 5, wherein the nebulizing system (30) comprises a reservoir (31) containing the composition comprising at least one molecule chosen from a non-volatile molecule and a temperature-unstable molecule (4), an extraction member (32) suitable for extracting the composition comprising at least one molecule chosen from a non-volatile molecule and a temperature-unstable molecule (4) from the reservoir (31), a nebulizing device (40) suitable for nebulizing said composition so as to form a stream of nebulized composition (5), the nebulizing device (40) comprising a nebulized composition dispensing member (42) suitable for directing and delivering said stream of nebulized composition to a nebulizing aperture (41).

7. The method for cosmetic treatment as claimed in claim 6, wherein the reservoir (31) is removably mounted on the extraction member (32), the method for cosmetic treatment providing for choosing a reservoir (31) among a range of reservoirs (31) comprising respectively different compositions, for mounting the reservoir (31) chosen on the extraction member (32), for nebulizing the composition contained in the reservoir (31) and for removing the reservoir (31) from the extraction member (32).

8. The method for cosmetic treatment as claimed in either one of claims 6 and 7, wherein the nebulizing device (40) comprises a component chosen from a piezoelectric grid and a spraying nozzle.

9. The method for cosmetic treatment as claimed in any one of claims 1 to 8, wherein the molecule chosen from a non-volatile molecule and a temperature-unstable molecule has a theoretical density, measured at 100°C and atmospheric pressure, greater than 0.9584.

10. The method for cosmetic treatment as claimed in any one of claims 1 to 9, wherein the molecule chosen from a non-volatile molecule and a temperature-unstable molecule has a molecular weight of between 50 and 2000 g/mol.

11. The method for cosmetic treatment as claimed in any one of claims 1 to 10, wherein the molecule chosen from a non-volatile molecule and an unstable molecule is chosen from:
- flavan-3-ols,
- group B vitamins (in particular B1, B3, B6, B9),
- vitamin C or ascorbic acid and derivatives thereof which are of glucoside type or which esterify an amino acid of lysine or proline type,
- vitamin E or tocopherol, or derivatives thereof, in particular phosphate or acetate derivatives,
- tocotrienols,
- alkaloids of xanthine, or methyl-, dimethyl- or trimethylxanthine type,
- temperature-unstable amino acids, in particular lysine, theanine, proline, tryptophan,
- lipids or fatty acids having at least one unsaturated double bond,
- sterols of cholesterol type or phytosterols and derivatives thereof,
- oligopeptides (up to a hexapeptide),
- glycols of the glycerol or propylene glycol type,
- polyholosides of hyaluronic acid type having a molecular weight of between 50 and 2000 g/mol,
- alpha- and beta-hydroxy acids, such as malic acid, citric acid or salicylic acid,
- and mixtures thereof.

12. The method for cosmetic treatment as claimed in claim 11, wherein the molecule chosen from a non-volatile molecule and an unstable molecule is a flavan-3-ol chosen from catechin, epicatechin, epicatechin gallate, epigallocatechin gallate, and mixtures thereof.

13. The method for cosmetic treatment as claimed in any one of claims 1 to 12, wherein the composition comprising at least one molecule chosen from a non-volatile molecule and a temperature-unstable molecule (4) has a viscosity of between 0.1 and 500 mPa.s, preferably 1 to 100 mPa.s, and more specifically between 1 and 50 mPa.s.

14. The method for cosmetic treatment as claimed in any one of claims 1 to 13, wherein the composition comprising at least one molecule chosen from a non-volatile molecule and a temperature-unstable molecule comprises any other constituent used in cosmetic compositions, chosen from water, a water-miscible organic solvent, oils, surfactants, fragrances, preservatives, neutralizing agents, sunscreens, moisturizing agents, self-tanning compounds, sequestrants, film-forming agents, and mixtures thereof.

15. The method for cosmetic treatment as claimed in any one of claims 1 to 14, wherein the composition comprising at least one molecule chosen from a non-volatile molecule and a temperature-unstable molecule (4) comprises one or more hydrophilic or lipophilic active agents chosen from antioxidants, moisturizing agents, emollients, anti-aging agents, anti-pollution agents, keratolytic agents, astringents, non-steroidal anti-inflammatories, whitening agents, and agents for combating aging.

16. The method for cosmetic treatment as claimed in any one of claims 1 to 15, providing for applying the composition comprising water (2) and the composition comprising at least one molecule chosen from a non-volatile molecule and a temperature-unstable molecule (4) to the face of a user.

17. The method for cosmetic treatment as claimed in any one of claims 1 to 16, providing for vaporizing the composition comprising water (2) and for nebulizing the composition comprising at least one molecule chosen from a non-volatile molecule and a temperature-unstable molecule (4) on a surface area (S) of between 300 cm² and 400 cm² located at a treatment distance (D) between 20 cm and 30 cm from the vaporizing system (10) and from the nebulizing system (30).

18. The method for cosmetic treatment as claimed in claim 17 when it is dependent on claims 5 and 6, wherein the stream of vapor (3) has a temperature of between 90°C and 110°C at the vaporizing aperture (21) and a temperature of between 35°C and 45°C at the treatment distance (D), and wherein the stream of nebulized composition (5) has a temperature of between 15°C and 30°C at the treatment distance (D).
